# EUROPEAN PATENT APPLICATION

(11) **EP 2 107 371 A1**
(43) Date of publication of application: **07.10.2009**
(21) Application number: 08703742.0
(22) Date of filing: 23.01.2008
(51) Int. Cl.: G01N 30/88, G01N 30/06, G01N 30/54, G01N 31/00

(54) **METHOD FOR SEPARATION OF ORGANIC HALOGEN, METHOD FOR MEASUREMENT OF CONCENTRATION OF LOW-VOLATILE ORGANIC HALOGEN, AND METHOD FOR MEASUREMENT OF CONCENTRATION OF DIOXIN**

(30) Priority: 25.01.2007 JP 2007014937
(71) Applicant: Electric Power Development Co., Ltd., Chuo-ku Tokyo 104-8165 (JP)
(72) Inventor: WATANABE, Gou, Tokyo 104-8165 (JP); MATSUDA, Tsuyoshi, Takehara-shi Hiroshima 729-2394 (JP); NAKUI, Hiroyuki, Chigasaki-shi Kanagawa 253-0041 (JP)
(74) Representative: Jump, Timothy John Simon
(86) International application number: PCT/JP2008/050912
(87) International publication number: WO 2008/090931

(57) **Abstract**

Disclosed is a method which can selectively separate organic halogens whose concentrations highly correlate with the concentration of dioxin in a gas to he measured such as an exhaust gas and, therefore, which can measure the concentrations of the organic halogens in the gas to determine the concentration of dioxin accurately. The method comprises the steps of: introducing a gas to he measured into a first or second adsorption column, where the gas is allowed to contact with an adsorbent having a surface area of 10 to 240 m /g at an adsorption temperature of 50 to 200°C to cause the selective adsorption of a low-volatile organic halogen among organic halogens contained in the gas: desorbing the low-volatile organic halogen from the adsorbent; decomposing the adsorbed gas on a thermal decomposition apparatus; measuring the concentration of a hydrogen halide contained in the decomposed gas on an analyzer; transmitting the measurement value to a data processing device to determine the concentration of the low-volatile organic halogen; and determining the concentration of dioxin based on the determined concentration of the low-volatile organic halogen.

## Description

### TECHNICAL FIELD

This invention relates to a method including selectively separating low-volatile organic halogens included in a gas to be measured such as exhaust gas from a combustion furnace or the atmosphere, measuring the concentration of these low-volatile organic halogens, and furthermore, rapidly and simply determining the concentration of dioxins in the gas to be measured from this concentration.
Priority is claimed on Japanese Patent Application No. 2007-014937, filed in Japan on January 25, 2007, the content of which is incorporated herein by reference.

### BACKGROUND ART OF INVENTION

The reaction in which dioxins are generated from unburned combustibles in the incineration exhaust gas of waste or the like with iron or copper in the soot and dust becoming a catalyst at a temperature range of 300 to 500°C is called de novo synthesis. It is considered that this de novo synthesis process is the main generation process of dioxins exhausted from a waste incineration process.

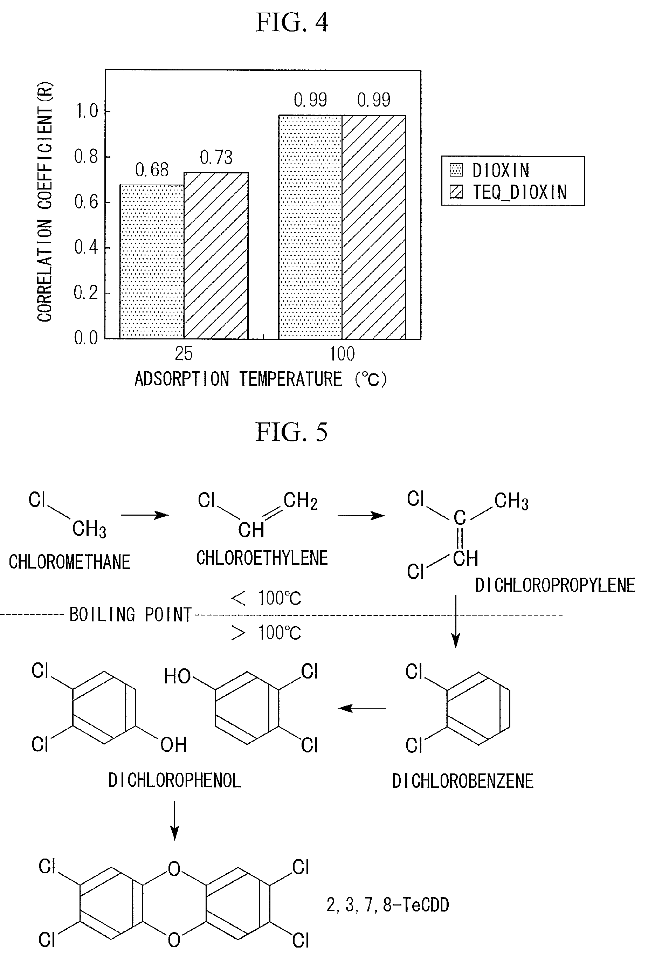

An example of such process is shown in FIG 5. It is considered that low-molecular unburned combustibles, such as chloromethane, repeatedly polymerize to become dioxins such as 2,3,7,8-TeCDD (2,3,7,8-tetrachlorodibenzo-dioxin).
Substances until they result in dioxins are called precursors and these are mostly organic halogens (mainly chlorinated products).

An official method for measuring the concentration of dioxins in exhaust gas is defined in JIS K0311. However, this measurement method requires about one month to determine the measurement results, and is thus completely unhelpful in the instantaneous measurement and continuous measurement of the concentration of dioxins in exhaust gas.

Rapid methods for measuring the concentration of dioxins in order to solve such problems are thus being examined. A method comprising determining the correlation between the concentration of organic halogens and the concentration of dioxins in a gas to be measured, and rapidly measuring the concentration of dioxins based on this correlation is being examined.
The present inventors have already proposed Japanese Patent No. 3458076 (Japanese Unexamined Patent Application, First Publication No. 2001-33433) as such a measurement method. This method extracts and quantifies all of the organic halogens, and can predict the concentration of dioxins from such values.

Also, Watanabe et al., in the measurement of organic halogens, have found that correlativity between low-volatile organic halogens and dioxins increases by selectively extracting low-volatile organic halogens using an adsorbent with a small surface area (10 m²/g) (Proceedings of the 13th Annual Conference of The Japan Society of Waste Management Experts 2002, pp. 772-774). The low-volatile organic halogens in this research were compounds with a carbon number of 12 to 20.

However, in the method of the research by Watanabe et al., the correlation coefficient of the correlativity between the concentration of low-volatile organic halogens and the concentration of dioxins is low at about 0.4 to 0.6, and thus there is the problem that the concentration of dioxins cannot be sufficiently predicted from the concentration of low-volatile organic halogens.
Patent Document 1: Japanese Patent No. 3458076 (Japanese Unexamined Patent Application, First Publication No. 2001-33433)
Non-patent Document 1: Proceedings of the 13th Annual Conference of The Japan Society of Waste Management Experts 2002, pp. 772-774

### DETAILED DESCRIPTION OF THE INVENTION

### PROBLEMS TO BE SOLVED BY THE INVENTION

Accordingly, the problems of the present invention are to be able to selectively separate organic halogens showing high concentration correlativity with the concentration of dioxins in a gas to be measured and to be able to accurately determine the concentration of dioxins by measuring the above-mentioned concentration of organic halogens in the gas to be measured.

### MEANS FOR SOLVING THE PROBLEMS

The present inventors have invented the following in order to solve such problems.
ITEM 1: A method for separating organic halogens comprising contacting a gas to be measured with an adsorbent at an adsorption temperature in a range of 50 to 200°C to cause the selective adsorption of low-volatile organic halogens among organic halogens contained in the gas to be measured and then, desorbing the low-volatile organic halogens from the adsorbent.

ITEM 2: The method for separating organic halogens disclosed in ITEM 1, wherein the boiling point of the above-mentioned low-volatile organic halogens is 100°C or more.

ITEM 3: The method for separating organic halogens disclosed in ITEM 1 or ITEM 2, wherein the desorption temperature is 300°C or more.

ITEM 4: The method for separating organic halogens disclosed in any one of ITEM 1 to ITEM 3, wherein the surface area of the above-mentioned adsorbent is in a range of 10 to 240 m²/g.

ITEM 5: A method for measuring the concentration of low-volatile organic halogens, comprising quantifying the concentration of the low-volatile organic halogens obtained by the separation method according to any one of ITEM 1 to ITEM 4.

ITEM 6: A method for measuring the concentration of dioxins comprising determining the correlation coefficient between the concentration of the low-volatile organic halogens obtained by the measurement method disclosed in ITEM 5 and a separately measured concentration of dioxins in the gas to be measured, and predicting the concentration of the dioxins in the gas to be measured from the above-mentioned concentration of low-volatile organic halogens based on this correlation coefficient.

### ADVANTAGEOUS EFFECTS OF THE INVENTION

In accordance with the present invention, by making the adsorption temperature 50 to 200°C, low-volatile organic halogens having a boiling point of 100°C or more in a gas to be measure are selective adsorbed. The correlativity between the concentration of these adsorbed low-volatile organic halogens and the concentration of dioxins in the gas to be measured is high and the correlation coefficient becomes about 0.99. It is thus possible to accurately know the concentration of dioxins by measuring the concentration of low-volatile organic halogens in the gas to be measured.
Accordingly, it is possible to rapidly and accurately determine the concentration of dioxins in the gas to be measured.

### BRIEF DESCRIPTION OF THE DRAWINGS

FIG. 1 is a schematic block diagram showing an example of a measurement device used in a separation measurement method of the present invention.
FIG. 2 is a diagram showing a relationship between the molecular weight and the boiling points of dioxins and precursors thereof.
FIG. 3 is a diagram showing the correlativity between the concentration of low-volatile organic halogens and the concentration of dioxins of the present invention.
FIG. 4 is a diagram showing the correlation coefficient of the concentration of low-volatile organic halogens and the concentration of dioxins of the present invention.
FIG. 5 is a diagram showing the de novo synthesis process of dioxins.

### DESCRIPTION OF THE REFERENCE SYMBOLS

1..MEASUREMENT DEVICE, 2..INORGANIC HALOGEN REMOVAL DEVICE, 3..FIRST SWITCHING VALVE, 4..FIRST ADSORPTION COLUMN, 5..SECOND ADSORPTION COLUMN, 6..SECOND SWITCHING VALVE, 7..SOURCE OF DESORPTION GAS, 8.. THERMAL DECOMPOSITION DEVICE, 9.. ANALYSIS DEVICE, 10..DATA PROCESSING DEVICE

### BEST MODE FOR CARRYING OUT THE INVENTION

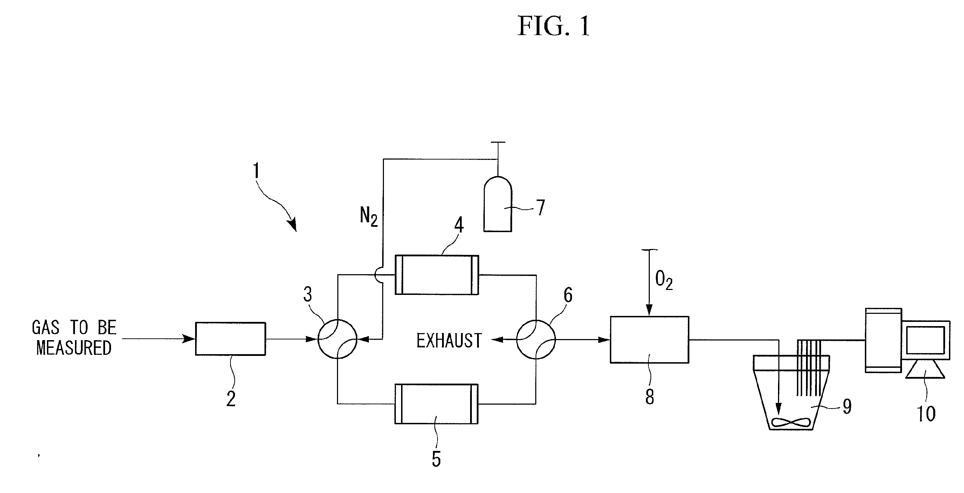

FIG. 1 shows an example of a measurement device used in a separation measurement method of the present invention.
A measurement device 1 sends a gas to be measured, dust and water having been removed in advance therefrom, to an inorganic halogen removal device 2. Here, inorganic halogens such as hydrogen chloride and chlorine gas for example are removed from the gas to be measured.
The inorganic halogen removal device 2 uses a removal tube filled with particles, the particles being plastic or ceramic and having metal particles, such as silver, with a particle size of about 0.4 to 1 mm or silver metal supported thereon or uses a removal tube filled with a filter.

The gas to be measured removed from the inorganic removal device 2 is sent to a first adsorption column 4 or a second adsorption column 5 via a first switching valve 3.
The first switching valve 3 is for switching the flow path of the gas to be measured so that the gas to be measured flows in the first adsorption column 4 or the second adsorption column 5 and to switch the flow path of an after-mentioned desorption gas so such flows in the first adsorption column 4 or in second adsorption column 5.

In the first adsorption column 4 or the second adsorption column 5, the surface area (value measured by BET) is preferably 10 to 240 m²/g and more preferably 90 to 110 m²/g of the carbon system. A graphite adsorbent is preferably filled. When the surface area of the adsorbent is less than 10 m²/g, it is difficult to adsorb low-volatile organic halogens and when the surface area of the adsorbent exceeds 240 m²/g, it is difficult to desorb the adsorbed low-volatile organic halogens. When the adsorbent is activated carbon or zeolite, the surface area thereof is too large and when the adsorbent is a resin, it is weak against heating.

The gas to be measured is introduced into the first adsorption column 4 and is adsorbed by the adsorbent. The adsorption temperature at this time is 50 to 200°C, preferably 90 to 110°C, and more preferably 100°C. In order to make the adsorption temperature within this temperature range, there is a method such as heaters for temperature adjustment are provided on the outside of adsorption columns 4, 5 and heated, the adsorbent is maintained in this temperature range, and the gas to be measured is warmed within this temperature range and introduced.

Among the organic halogens in the gas to be measured, low-volatile organic halogens are selectively adsorbed under such adsorption temperature conditions, and the high-volatile organic halogens are not adsorbed and are exhausted to the outside of the system as is from the first adsorption column 4 via the second switching valve 6.
In this invention, low-volatile organic halogens refer to organic halogens having a boiling point of 100°C or more and 450°C or less, and high-volatile organic halogens refer to organic halogens having a boiling point of -24°C or more and less than 100°C.

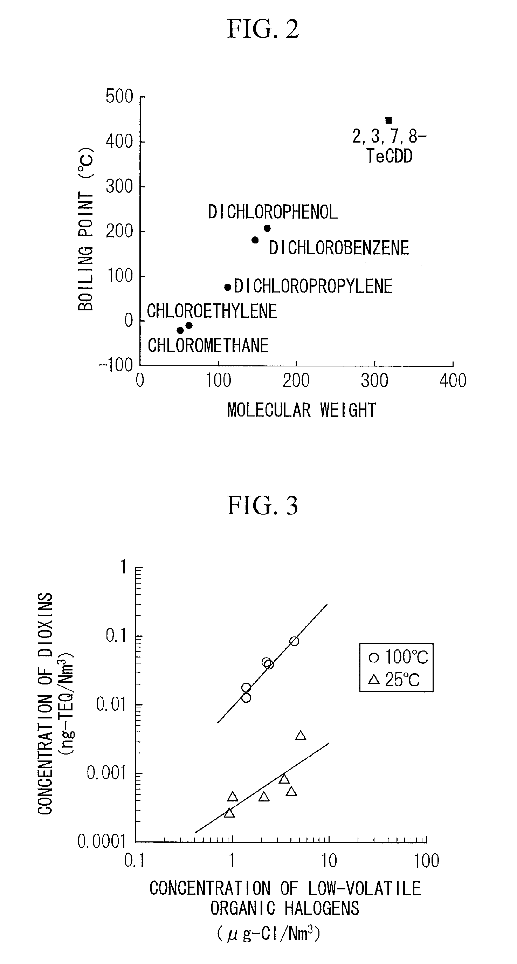

FIG. 2 shows a relationship between the molecular weight and the boiling points of dioxins and precursors thereof. When the de novo synthesis shown in the above-mentioned FIG. 5 proceeds, chain-like organic halogens form benzene rings and in accordance with this, the molecular weight increases, thus increasing the boiling point to exceeding 100°C.

When the adsorption temperature is less than 50°C, high-volatile organic halogens are not vaporized and are adsorbed by the adsorbent. Furthermore, it is possible that moisture remaining in the gas to be measured condensates and is adsorbed by the adsorbent.
When the adsorption temperature exceeds 200°C, high-volatile organic halogens are vaporized and insufficiently adsorbed by the adsorbent, thus leading to a reduction in yield.

Just before saturation of the adsorbent in the first adsorption column 4, the first and second switching valves 3, 6 are switched, causing the gas to be measured to flow in the second adsorption column 5. A desorption gas such as nitrogen, argon, or helium is sent from a desorption gas source 7 to the first adsorption column 4 via the first switching valve 3 and the low-volatile organic halogens adsorbed here are desorbed from the adsorbent.

The desorption temperature at this time is 300°C or more, preferably 300°C to 500°C, and more preferably 400 to 450°C. When less than 300°C, desorption of the low-volatile organic halogens is insufficient and when exceeding 500°C, the adsorbent deteriorates. Also, the boiling point of the dioxins is about this temperature range and is the temperature for which most of the adsorbed low-volatile organic halogens vaporize.

The desorption gas from the first adsorption column 4 is sent to a thermal decomposition device 8. Here, an oxygen-containing gas such as oxygen gas or air is added and the desorption gas is thermally decomposed.
The thermal decomposition device 8 includes a combustion tube formed from silica or the like and by a heater provided on the outside of this combustion tube, heats to about 700 to 1,000°C the mixture gas of desorption gas and oxygen-containing gas sent to inside the tube to combust the same. The low-volatile organic halogens in the desorption gas are thus decomposed to become hydrogen halides, carbon dioxide, water, and the like.

The thermally decomposed gas from the thermal decomposition device 8 is sent to an analysis device 9. Here, the concentration of hydrogen halides in the thermally decomposed gas is quantitatively analyzed.
A gas chromatograph, a liquid chromatograph, a coulometric titration analysis device, a conductometry device, an ion electrode analysis device, an infrared spectroscope, a plasma emission spectrometer, or the like can be used as the analysis device 9. Among these, a coulometric titration analysis device conforming to ASTM D5808 is preferred.
Also, with the coulometric titration analysis device, it is not necessary to prepare a calibration curve for analysis, and thus quick and automatic quantitative determination with high accuracy is easily possible.

The measured value of the concentration of hydrogen halides measured by the analysis apparatus 9 is sent to a data processing device 10. Here, based on the concentration of hydrogen halides, the concentration of low-volatile organic halogens is calculated and furthermore, the concentration of dioxins is calculated.

Just before saturation of the adsorbent in the second adsorbent column 5, the first and second switching valves 3, 6 are switched, causing the desorption gas to flow in the second adsorption column 5, and by carrying out a similar operation, the gas to be measured again flows in the first adsorption column 4.
By repeating that same operations, the concentration of the dioxins in the gas to be measured can be continuously measured.

In such an analytical measurement method, the correlation between the concentration of the low-volatile organic halogens in the gas to be measured and the concentration of dioxins in the gas to be measured is very high.
FIG. 3 shows the correlation between the concentration of low-volatile organic halogens quantified by adsorption and analysis of the gas to be measured at 25°C and 100°C and the concentration of dioxins by the official method defined in JIS K0311 in the same gas to be measured. In FIG 3, TEQ is the amount of the measured dioxins represented in terms of toxicity of 2,3,7,8-TeCDD, which is the most toxic. Also, Cl represents chlorine and N represents the standard state.

Here, exhaust gas from a general waster incineration facility was used as the gas to be measured, a graphite adsorbent having a surface area of 100 m²/g was used as the adsorbent, nitrogen was used as the desorption gas, and was carried out at a desorption temperature of 450°C.
From the graph of FIG. 3, although a positive correlation was seen between the low-volatile organic halogens and the concentration of the dioxins under either adsorption temperature conditions, variation in correlation was smaller at 100°C compared to at 25°C.

Also, the concentration of dioxins with respect to the low-volatile organic halogens was ten or more times larger at adsorption at 100°C compared to adsorption at 25°C. This shows that that the proportion of dioxins in the organic halogens is 10 or more times higher when adsorbed at 100°C compared to when adsorbed at 25°C.

FIG 4 shows the results of regression analysis of the results shown in FIG. 3 to determine the correlation coefficient (R). In FIG. 4, TEQ_dioxin represents the situation when the amount of measured dioxins is represented in terms of toxicity of 2,3,7,8-TeCDD, which is the most toxic. From FIG. 4, although R of organic halogens and dioxins adsorbed at 25°C was 0.68 to 0.73, the correlation coefficient was 0.99 when adsorbed at 100°C. This shows that adsorption at 100°C has higher correlation than adsorption at 25°C and thus means that it is more effective as an alternate index of dioxins.

As above-mentioned, by separating the low-volatile organic halogens in a gas to be measured and quantifying the concentration thereof by this separation measurement method, the concentration of dioxins in the gas to be measured can be accurately predicted.
Furthermore, by a simple operation in a short period of time, this method makes it possible to understand the concentration of dioxins at an accuracy equivalent to the current official method without having to use such method.

### INDUSTRIAL APPLICABILITY

In accordance with the present invention, by making the adsorption temperature 50 to 200°C, low-volatile organic halogens having a boiling point of 100°C or more in a gas to be measure are selective adsorbed. The correlativity between the concentration of these adsorbed low-volatile organic halogens and the concentration of dioxins in the gas to be measured is high. It is thus possible to accurately know the concentration of dioxins by measuring the concentration of low-volatile organic halogens in the gas to be measured.
Accordingly, it is possible to rapidly and accurately determine the concentration of dioxins in the gas to be measured. Therefore, the present invention is industrially useful.

## Claims

1. A method for separating organic halogens comprising the steps of:
contacting a gas to be measured with an adsorbent at an adsorption temperature in range of 50 to 200°C to cause the selective adsorption of low-volatile organic halogens among organic halogens contained in the gas to be measured; and
desorbing the low-volatile organic halogens from the adsorbent.

2. The method for separating organic halogens according to claim 1, wherein the boiling point of the above-mentioned low-volatile organic halogens is 100°C or more.

3. The method for separating organic halogens according to claim 1, wherein the desorption temperature is 300°C or more.

4. The method for separating organic halogens according to claim 1, wherein the surface area of the above-mentioned adsorbent is in a range of 10 to 240 m²/g.

5. A method for measuring the concentration of low-volatile organic halogens, comprising quantifying the concentration of the low-volatile organic halogen obtained by the separation method according to claim 1.

6. A method for measuring the concentration of dioxins comprising the steps of:
determining a correlation coefficient between the concentration of the low-volatile organic halogens obtained by the measurement method according to claim 5 and a separately measured concentration of dioxins in the gas to be measured; and
predicting the concentration of the dioxins in the gas to be measured from the concentration of low-volatile organic halogens based on the correlation coefficient.
